(19) Europäisches Patentamt
European Patent Office
Office européen des brevets

(11) **EP 3 219 697 B1**

(12) **EUROPÄISCHE PATENTSCHRIFT**

(45) Veröffentlichungstag und Bekanntmachung des Hinweises auf die Patenterteilung:
**13.06.2018 Patentblatt 2018/24**

(51) Int Cl.:
***C07C 29/151*** *(2006.01)*

(21) Anmeldenummer: **16400006.9**

(22) Anmeldetag: **16.03.2016**

(54) **METHANOLSYNTHESE AUS SYNTHESEGASEN MIT WASSERSTOFFMANGEL**

THE SYNTHESIS OF METHANOL FROM SYNTHESIS GASES WITH HYDROGEN MANGLE

SYNTHESE DE METHANOL A PARTIR DE GAZ DE SYNTHESE AYANT UN MANQUE D'HYDROGENE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**20.09.2017 Patentblatt 2017/38**

(73) Patentinhaber: **L'Air Liquide Société Anonyme pour l'Etude et l'Exploitation des Procédés Georges Claude 75007 Paris (FR)**

(72) Erfinder:
• **Kopetsch, Hans**
 **61352 Bad Homburg (DE)**

• **Gronemann, Veronika**
 **61184 Karben (DE)**
• **Oelmann, Tobias**
 **61118 Bad Vilbel (DE)**

(74) Vertreter: **Dropsch, Holger**
 **Air Liquide Forschung und Entwicklung GmbH Gwinnerstraße 27-33 60388 Frankfurt am Main (DE)**

(56) Entgegenhaltungen:
 **EP-B1- 0 790 226     US-A1- 2009 018 220**

**Beschreibung**

**Gebiet der Erfindung**

Die vorliegende Erfindung betrifft ein Verfahren und eine Anlage zur Methanolsynthese, insbesondere zur Methanolsynthese aus einem Synthesegas, das einen Wasserstoffmangel aufweist.

**Stand der Technik**

Verfahren zur Herstellung von Methanol durch katalytische Umsetzung von Wasserstoff und Kohlenstoffoxide enthaltendem Synthesegas sind der Fachwelt seit langer Zeit bekannt. So werden in Ullmann's Encyclopedia of Industrial Chemistry, Sixth Edition, 1998 Electronic Release, Kapitel "Methanol", Unterkapitel 5.2 "Synthesis" verschiedene Grundverfahren zur Herstellung von Methanol beschrieben.

Ein moderneres, zweistufiges Verfahren zur Herstellung von Methanol ist beispielsweise aus der EP 0 790 226 B1 bekannt. Methanol wird demnach in einem Kreisprozess hergestellt, bei dem eine Mischung von frischem und teilweise reagiertem Synthesegas zunächst einem wassergekühlten Reaktor und dann einem gasgekühlten Reaktor zugeführt wird, in welchen das Synthesegas jeweils an einem kupferbasierten Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280°C vorgewärmt wird, bevor es in den ersten Synthesereaktor eingebracht wird. Ein Teil des zurückzuführenden Synthesegases wird als Spülstrom (sog. Purge) aus dem Verfahren entfernt, der gegenüber dem im System vorhandenen Gasinventar klein ist, um zu verhindern, dass sich Inertkomponenten, Verunreinigungen oder Nebenprodukte innerhalb des Synthesegaskreislaufes anreichern. Diese Maßnahme wird auch in der deutschen Offenlegungsschrift DE 2934332 A1 und der europäischen Patentanmeldung EP 1016643 A1 gelehrt.

Dieses Verfahren setzt allerdings voraus, das die Stöchiometriezahl (R) des verwendeten Synthesegases, definiert durch die Formel:

$$R = ([H_2] - [CO_2]) / ([CO] + [CO_2]),$$

mit [x] = Konzentration der Komponente x,
mindestens 2 beträgt, dass das verwendete Synthesegas im Hinblick auf die Herstellung von Methanol also ausreichend Wasserstoff enthält. Synthesegase mit Wasserstoffmangel können dagegen aus Reformierverfahren erhalten werden, die eine Stufe der Teiloxidation beinhalten, wie beispielsweise dem autothermen Reformieren (ATR). In einem derartigen Fall wird der Wasserstoff in der Methanolsynthesereaktion verbraucht, während ein wesentlicher Anteil der Kohlenoxide unumgesetzt zurückbleibt, was zu einer Zusammensetzung in der Syntheseschleife führt, die einen hohen Anteil an Kohlenoxiden aufweist, jedoch arm an Wasserstoff ist, also ein Wasserstoffdefizit oder einen Wasserstoffmangel aufweist. Das hat verschiedene Konsequenzen, zu denen gehört, dass das erforderliche Katalysatorvolumen hoch ist und dass der Anteil an Nebenprodukten (insbesondere höhere Alkohole und Ketone) deutlich höher ist als normal.

Es ist an sich bekannt, dem $H_2$-defizitären Synthesegas Wasserstoff aus anderen Quellen zuzuführen, um die Stöchiometriezahl in den optimalen Bereich von 2 oder höher zu bringen.

Als Wasserstoffquelle kann dabei unter anderem der Spülstrom dienen, der noch einen Anteil nicht umgesetzten Wasserstoffs enthält, der mittels einer Wasserstoffgewinnungseinheit abgetrennt werden kann. Die typische Wasserstoffkonzentration im Spülstrom beträgt rund 70 Vol.-%. Eine andere Alternative besteht darin, aus einem Seitenstrom des frischen Synthesegases, das auch als Frischgas (Make-up-Gas, MUG) bezeichnet wird, Wasserstoff zu gewinnen und diesen Wasserstoff zurück in das Synthesegas einzuspeisen. So lehrt die offengelegte US-Patentanmeldung US 2009/0018220 A1 ein Methanolsyntheseverfahren, bei dem aus wenigstens einem Teil des genannten Spülgases und einem Teil des genannten Frischgases Wasserstoff gewonnen wird, Wobei der (rück)gewonnene Wasserstoff in die Synthesegasmischung eingeführt wird. Als dabei verwendete Wasserstoffgewinnungseinheiten wird die Druckwechseladsorption (pressure swing adsorption, PSA) oder - als Alternative dazu - die $H_2$-Abtrennung mittels Membran genannt.

Der Nachteil dieser Anordnung besteht jedoch darin, dass innerhalb der Wasserstoffgewinnungseinheit ein Teil des Wasserstoffs verloren geht, bevor er die Syntheseschleife überhaupt erreicht. Wenn zudem bereits der Synthesegas-Frischgasstrom ein Wasserstoffdefizit enthält, wird dieses durch zusätzliche $H_2$-Abtrennung aus einem Frischgas-Seitenstrom nur noch verstärkt. Durch die $H_2$-Abtrennung aus dem Frischgas-Seitenstrom wird dann in der Wasserstoffgewinnungseinheit ein CO und $CO_2$ enthaftender Abfallstrom erhalten, wobei die dort enthaltenen Kohlenoxide der Methanolsynthese verlorengehen.

**Beschreibung der Erfindung**

Es ist daher die Aufgabe der vorliegenden Erfindung, ein Methanol-Syntheseverfahren bereitzustellen, das in der Lage ist, Synthesegas mit einem Wasserstoffmangel zu verarbeiten, ohne dabei die beschriebenen Nachteile des Standes der Technik aufzuweisen.

Diese Aufgabe wird durch ein Verfahren mit den Merkmalen des Anspruchs 1 und durch eine Anlage mit den Merkmalen des Anspruchs 11 gelöst. Weitere Ausgestaltungen der Erfindung ergeben sich aus den jeweiligen Unteransprüchen.

Erfindungsgemäßes Verfahren:

Verfahren zur Herstellung von Methanol aus einem Wasserstoff, Kohlenstoffoxide und optional Inertkomponenten enthaltenden Synthesegas, umfassend folgende Schritte:

(a) Bereitstellen eines Wasserstoff, Kohlenstoffoxide und optional Inertkomponenten enthaltenden Synthesegas-Frischgasstroms, der bezüglich der für die Methanolsynthese vorgegebenen Stöchiometrie ein Wasserstoffdefizit enthält,

(b) Zusammenführen des an Wasserstoff defizitären Synthesegas-Frischgasstroms mit einem Wasserstoff enthaltenden Trimmgasstrom zu einem an Wasserstoff angereicherten Synthesegas-Frischgasstrom,

(c) Zusammenführen des an Wasserstoff angereicherten Synthesegas-Frischgasstroms mit dem in Schritt (g) erhaltenen Synthesegas-Kreisiaufgasstrom zu einem Synthesegas-Feedgasstrom,

(d) Einleiten des Synthesegas-Feedgasstroms in mindestens einen Methanolsynthesereaktor und mindestens teilweises, katalytisches Umsetzen der in dem Synthesegas-Feedgasstrom enthaltenen Kohlenstoffoxide mit Wasserstoff unter Methanolsynthesebedingungen zu Methanol,

(e) Ausleiten eines Methanoldampf und nicht umgesetzte Synthesegasbestandteile enthaltenden Produktgasstroms aus dem mindestens einen Methanolsynthesereaktor,

(f) Mindestens teilweises Abtrennen des Methanols aus dem Produktgasstrom durch Abkühlen und Kondensation,

(g) Auftrennen des Produktgasstroms nach Abtrennen des Methanols in einen Synthesegas-Kreislaufgasstrom und einen mit Methanolresten beladenen Spülgasstrom, Rückführen des Synthesegas-Kreislaufgasstroms nach Schritt (c),

(h) Einleiten des mit Methanolresten beladenen Spülgasstroms in eine Waschvorrichtung, Inkontaktbringen des mit Methanolresten beladenen Spülgasstroms in der Waschvorrichtung mit einem Waschmittel, bevorzugt Wasser, Ausleiten eines an Methanol abgesicherten Spülgasstroms und eines mit Methanol beladenen Waschmittels aus der Waschvorrichtung,

(i) Einleiten des an Methanol abgereicherten Spülgasstroms in eine Wasserstoffabtrennungsvorrichtung, die eine Membrantrennstufe und eine Druckwechseladsorptionsstufe umfasst, wobei mindestens ein an Wasserstoff angereicherter und mindestens ein an Wasserstoff abgereicherter Gasstrom erhalten wird,

(j) Rückführen mindestens eines an Wasserstoff angereicherten Gasstroms als Wasserstoff enthaltender Trimmgasstrom nach Schritt (b).

Erfindungsgemäße Anlage:

Anlage zur Herstellung von Methanol aus einem Wasserstoff, Kohlenstoffoxide und optionalInertkomponenten enthaltenden Synthesegas, umfassend folgende Anlagenkomponenten:

(a) eine Synthesegaserzeugungsstufe zum Bereitstellen eines Wasserstoff, Kohlenstoffoxide und optional Inertkomponenten enthaltenden Synthesegas-Frischgasstroms, der bezüglich der für die Methanolsynthese vorgegebenen Stöchiometrie ein Wasserstoffdefizit enthält,

(b) eine erste Mischvorrichtung zum Zusammenführen des an Wasserstoff defizitären Synthesegas-Frischgasstroms mit einem Wasserstoff enthaltenden Trimmgasstrom zu einem an Wasserstoff angereicherten Synthesegas-Frischgasstrom,

(c) eine zweite Mischvorrichtung zum Zusammenführen des an Wasserstoff angereicherten Synthesegas-Frischgasstroms mit einem Synthesegas-Kreislaufgasstrom zu einem Synthesegas-Feedgasstrom,

(d) mindestens einen Methanolsynthesereaktor, eine Leitung zum Einleiten des Synthesegas-Feedgasstroms in den mindestens einen Methanolsynthesereaktor,

(e) eine Leitung zum Ausleiten eines Methanoldampf und nicht umgesetzte Synthesegasbestandteile enthaltenden Produktgasstroms aus dem mindestens einen Methanolsynthesereaktor,

(f) eine Abtrennvorrichtung zum mindestens teilweisen Abtrennen des Methanols aus dem Produktgasstrom durch Abkühlen und Kondensation,

(g) eine Auftrennvorrichtung zum Auftrennen des Produktgasstroms nach Abtrennen des Methanols in einen Synthesegas-Kreislaufgasstrom und einen mit Methanolresten beladenen Spülgasstrom, sowie eine Leitung zum Rückführen des Synthesegas-Kreislaufgasstroms zu der zweiten Mischvorrichtung,

(h) eine Waschvorrichtung, eine Leitung zum Einleiten des mit Methanolresten beladenen Spülgasstroms in die Waschvorrichtung, eine Leitung zum Einleiten des Waschmittels, eine Leitung zum Ausleiten eines an Methanol abgereicherten Spülgasstroms aus der Waschvorrichtung und eine Leitung zum Ausleiten eines mit Methanol beladenen Waschmittels aus der Waschvorrichtung,

(i) eine Wasserstoffabtrennungsvorrichtung, umfassend eine Membrantrennstufe und eine Druckwechseladsorptionsstufe, eine Leitung zum Einleiten des an Methanol abgereicherten Spülgasstroms in die Wasserstoffabtrennungsvorrichtung, eine Leitung zum Ausleiten eines an Wasserstoff angereicherten Gasstroms aus der Wasserstoffabtrennungsvorrichtung und eine Leitung zum Ausleiten eines an Wasserstoff abgereicherten Gasstroms aus der Wasserstoffabtrennungsvorrichtung,

(j) eine Leitung zum Rückführen eines an Wasserstoff angereicherten Gasstroms als Wasserstoff enthaltender Trimmgasstrom zu der ersten Mischvorrichtung.

Die für die Umsetzung von Synthesegas zu Methanol erforderlichen Umsetzungsbedingungen bzw. Methanolsynthesebedingungen sind dem Fachmann aus dem Stand der Technik, beispielsweise den eingangs erörterten Druckschriften, bekannt. Notwendige Anpassungen dieser Bedingungen an die jeweiligen Betriebserfordernisse wird er auf der Grundlage von Routineversuchen vornehmen.

Unter Fluidverbindung zwischen zwei Bereichen der erfindungsgemäßen Anlage wird dabei jegliche Art von Verbindung verstanden, die es ermöglicht, dass ein Fluid, beispielsweise ein Gasstrom, von dem einen zu dem anderen der beiden Bereiche strömen kann, unbeachtlich etwaiger zwischengeschalteter Bereiche oder Bauteile.

Das als Waschmittel verwendete Wasser ist zumeist demineralisiertes Wasser. Es können aber auch andere, insbesondere Wasserqualitäten höherer Reinheit, beispielsweise Reinstwasser oder destilliertes Wasser, als Waschmittel eingesetzt werden. Wasser geringerer Reinheit ist dann als Waschmittel einsetzbar, wenn die vorhandenen Begleitstoffe in nachgeschalteten Verfahrensstufen keine Probleme bereiten.

Die Verwendung einer Wasserstoffabtrennungsvorrichtung, umfassend eine Membrantrennstufe und eine Druckwechseladsorptionsstufe, ermöglicht eine effiziente Abtrennung des zur Einstellung der gewünschten Stöchiometriezahl benötigten Wasserstoffs.

Die dabei anfallenden Abfallströme können weiter verwendet werden, beispielsweise als Brenngas innerhalb der der Methanolsynthese vorgeschalteten Reformeranlage oder als Heizgas bzw. Brenngas in einer Vorrichtung zur Dampferzeugung. Auch eine stoffliche Nutzung der Abfallströme, beispielsweise in einer benachbarten Anlage zur Kohlevergasung, ist möglich. Die erfindungsgemäße Befreiung des Spülgasstroms von Methanolresten in der Waschvorrichtung wirkt in vorteilhafter Weise mit der Wasserstoffabtrennungsvorrichtung zusammen, da deren Standzeit erhöht wird: Methanolreste im Spülgas würden ansonsten die Membran der Membrantrennstufe schädigen bzw. einen Teil der Adsorptionskapazität des in der Druckwechseladsorptionsstufe eingesetzten Adsorbens belegen.

Die Kombination von Membrantrennung und Druckwechseladsorption (PSA) führt zu einer technisch und ökonomisch sinnvollen Wasserstoffabtrennung: Der Einsatz einer PSA führt zu hochreinem Wasserstoff (Reinheit 99,9 Vol.-%) bei einer Rückgewinnung von ca. 80 % des Wasserstoffes in dem zu trennenden Gas. Allerdings handelt es sich hierbei um eine relativ komplizierte und kostenintensive Technik. Andererseits führt die Membrantrennung zu einer geringeren Reinheit des abgetrennten Wasserstoffs (ca. 80 bis 90 Vol.-% im Permeat) bei einer Rückgewinnung von ca. 70 % des Wasserstoffs aus dem Spülgas, wobei es sich hierbei aber um eine relativ einfache und preiswerte Technik handelt.

Eine Kopplung der genannten Trennverfahren führt daher zur optimalen Nutzung der Vorteile und Einsparung von Kosten, sowie zu hoher Flexibilität hinsichtlich der Anwendung: Wenn die rückzuführende Wasserstoffmenge maximiert werden soll, kommt eine serielle Verschaltung der Membrantrennstufe mit nachgeschalteter PSA in Betracht. Wenn dagegen ein Teil des abgetrennten, hochreinen Wasserstoffs an Verbraucher außerhalb des Verfahrens exportiert werden soll, kann eine Parallelschaltung von Membrantrennstufe und PSA sinnvoll sein. Der hochreine Wasserstoff wird dann ausschließlich aus der PSA abgenommen und an den externen Verbraucher abgegeben.

**Bevorzugte Ausgestaltungen der Erfindung**

In einer bevorzugten Ausgestaltung des erfindungsgemäßen Verfahrens sind in der Wasserstoffabtrennungsvorrichtung die Membrantrennstufe und die Druckwechseladsorptionsstufe hintereinander geschaltet und stehen in Fluidverbindung miteinander. Wie bereits oben erläutert, empfiehlt sich diese Verfahrensführung, wenn die rückzuführende Wasserstoffmenge maximiert werden soll.

Eine weitere bevorzugte Ausgestaltung des erfindungsgemäßen Verfahrens ist dadurch gekennzeichnet, dass in der Wasserstoffabtrennungsvorrichtung zunächst die Membrantrennstufe und anschließend die Druckwechseladsorptionsstufe von dem an Methanol abgereicherten Spülgasstrom durchströmt werden. Auf diese Weise erfolgt eine Vorabtrennung in der vergleichsweise unselektiv arbeitenden Membrantrennstufe und die Feintrennung in der hochselektiv arbeitenden Druckwechseladsorptionsstufe, wobei letztere dann nur mit einem kleineren Volumenstrom belastet wird und somit kleiner ausgelegt werden kann.

In Weiterbildung der zuvor erörterten Ausgestaltung des erfindungsgemäßen Verfahrens wird in der Membrantrennstufe als Permeat ein erster an Wasserstoff angereicherter Gasstrom gewonnen wird, der als Trimmgasstrom mit dem an Wasserstoff defizitären Synthesegas-Frischgasstrom zusammengeführt wird. Die relativ geringe Wasserstoffreinheit im Permeat reicht oft bereits für eine Einstellung der erwünschten Stöchiometriezahl im Synthesegas-Feedgasstrom aus, insbesondere dann, wenn das Wasserstoffdefizit nur gering ist.

In weiterer Weiterbildung der zuvor erörterten Ausgestaltung des erfindungsgemäßen Verfahrens wird das in der Membrantrennstufe erhaltene Retentat der Druckwechseladsorptionsstufe aufgegeben und in der Druckwechseladsorptionsstufe ein zweiter an Wasserstoff angereicherter Gasstrom gewonnen, der als Trimmgasstrom mit dem an Wasserstoff defizitären Synthesegas-Frischgasstrom zusammengeführt wird oder als Wasserstoff-Exportstrom aus dem Verfahren ausgeleitet wird. Eine Verwendung als weiterer Trimmgasstrom bringt vor allem dann Vorteile, wenn der Synthesegas-Feedgasstrom ein größeres Wasserstoffdefizit aufweist. Ansonsten kann in der in der Druckwechseladsorptionsstufe erhaltene, an Wasserstoff angereicherte Gasstrom als Wasserstoff-Exportstrom an externe Verbraucher abgegeben werden.

Besonders bevorzugt wird es bei der zuvor erörterten Ausgestaltung des erfindungsgemäßen Verfahrens, wenn der zweite an Wasserstoff angereicherte Gasstrom zuerst mit dem ersten an Wasserstoff angereicherten Gasstrom und anschließend als Trimmgasstrom mit dem an Wasserstoff defizitären Synthesegas-Frischgasstrom zusammengeführt wird. Auf diese Weise wird eine homogene Beschaffenheit des Trimmgasstroms und des an Wasserstoff angereicherten Synthesegas-Frischgasstroms erreicht.

In alternativer Ausgestaltung des erfindungsgemäßen Verfahrens sind in der Wasserstoffabtrennungsvorrichtung die Membrantrennstufe und die ruckwechseladsorptionsstufe parallel geschaltet, wobei ein Teil des an Methanol abgereicherten Spülgasstroms der Membrantrennstufe und der verbliebene Teil des an Methanol abgereicherten Spülgasstroms der Druckwechseladsorptionsstufe aufgegeben wird. Wie bereits oben erläutert, empfiehlt sich diese Verfahrensführung, wenn ein Teil des mittels PSA abgetrennten, hochreinen Wasserstoffs an Verbraucher außerhalb des Verfahrens exportiert werden soll.

In Weiterbildung der zuvor erörterten Ausgestaltung des erfindungsgemäßen Verfahrens wird in der Membrantrennstufe als Permeat ein erster an Wasserstoff angereicherter Gasstrom gewonnen, der als Trimmgasstrom mit dem an Wasserstoff defizitären Synthesegas-Frischgasstrom zusammengeführt wird. Die relativ geringe Wasserstoffreinheit im Permeat reicht oft bereits für eine Einstellung der erwünschten Stöchiometriezahl im Synthesegas-Feedgasstrom aus, insbesondere dann, wenn das Wasserstoffdefizit nur gering ist.

In weiterer Ausgestaltung des erfindungsgemäßen Verfahrens wird in der Druckwechseladsorptionsstufe ein zweiter an Wasserstoff angereicherter Gasstrom gewonnen, der als Trimmgasstrom mit dem an Wasserstoff defizitären Synthesegas-Frischgasstrom zusammengeführt wird oder als Wasserstoff-Exportstrom aus dem Verfahren ausgeleitet wird. Eine Verwendung als weiterer Trimmgasstrom bringt vor allem dann Vorteile, wenn der Synthesegas-Feedgasstrom ein größeres Wasserstoffdefizit aufweist. Ansonsten kann in der in der Druckwechseladsorptionsstufe erhaltene, an Wasserstoff angereicherte Gasstrom als Wasserstoff-Exportstrom an externe Verbraucher abgegeben werden.

In einem weiteren Aspekt der Erfindung erfolgt das Bereitstellen des Synthesegas-Frischgasstroms mittels einer Synthesegaserzeugungsstufe, die einen Autothermreformer umfasst. Der auf diese Weise erhaltene Synthesegas-Frischgasstrom weist oft ein Wasserstoffdefizit auf, da aufgrund der oxidativen Reaktionsbedingungen im Autothermreformer ein Teil des gebildeten Wasserstoffs durch Verbrennen verlorengeht.

In besonderer Ausgestaltung der erfindungsgemäßen Anlage ist diese dadurch gekennzeichnet, dass in der Wasserstoffabtrennungsvorrichtung die Membrantrennstufe und die Druckwechseladsorptionsstufe hintereinander geschaltet sind und in Fluidverbindung miteinander stehen. Wie bereits oben erläutert, empfiehlt sich diese Verfahrensführung, wenn die rückzuführende Wasserstoffmenge maximiert werden soll.

Eine weitere bevorzugte Ausgestaltung der erfindungsgemäßen Anlage ist dadurch gekennzeichnet, dass in der Wasserstoffabtrennungsvorrichtung zunächst die Membrantrennstufe und anschließend die Druckwechseladsorptionsstufe von dem an Methanol abgereicherten Spülgasstrom durchströmt werden. Auf diese Weise erfolgt eine Vorabtrennung in der vergleichsweise unselektiv arbeitenden Membrantrennstufe und die Feintrennung in der hochselektiv arbeitenden Druckwechseladsorptionsstufe, wobei letztere dann nur mit einem kleineren Volumenstrom belastet wird und somit kleiner ausgelegt werden kann.

In Weiterbildung der zuvor erörterten Ausgestaltung der erfindungsgemäßen Anlage umfasst diese auch eine Leitung, mit der ein erster an Wasserstoff angereicherter Gasstrom, der in der Membrantrennstufe als Permeat gewonnen wird, als Trimmgasstrom zu der ersten Mischvorrichtung zurückgeführt wird. Die relativ geringe Wasserstoffreinheit im Permeat reicht oft bereits für eine Einstellung der erwünschten Stöchiometriezahl im Synthesegas-Feedgasstrom aus, insbesondere dann, wenn das Wasserstoffdefizit nur gering ist.

In weiterer Weiterbildung der zuvor erörterten Ausgestaltung der erfindungsgemäßen Anlage umfasst diese auch eine Leitung zum Einleiten des in der Membrantrennstufe erhaltenen Retentats in die Druckwechseladsorptionsstufe und eine Leitung zum Einleiten eines zweiten an Wasserstoff angereicherten Gasstroms, der in der Druckwechseladsorptionsstufe gewonnen wird, als Trimmgasstrom in die erste Mischvorrichtung oder zum Ausleiten aus der Anlage als Wasserstoff-Exportstrom. Eine Verwendung als weiterer Trimmgasstrom bringt vor allem dann Vorteile, wenn der Synthesegas-Feedgasstrom ein größeres Wasserstoffdefizit aufweist. Ansonsten kann in der in der Druckwechseladsorptionsstufe erhaltene, an Wasserstoff angereicherte Gasstrom als Wasserstoff-Exportstrom an externe Verbraucher abgegeben werden.

Besonders bevorzugt wird es bei der zuvor erörterten Ausgestaltung der erfindungsgemäßen Anlage, wenn sie ferner eine Mischvorrichtung zum Zusammenführen des zweiten an Wasserstoff angereicherten Gasstroms mit dem ersten an Wasserstoff angereicherter Gasstrom, sowie eine Leitung zum Zuführen des dabei erhaltenen Mischgasstroms zu der ersten Mischvorrichtung umfasst. Auf diese Weise wird eine homogene Beschaffenheit des Trimmgasstroms und des an Wasserstoff angereicherten Synthesegas-Frischgasstroms erreicht.

In alternativer Ausgestaltung der erfindungsgemäßen Anlage sind in der Wasserstoffabtrennungsvorrichtung die Membrantrennstufe und die Druckwechseladsorptionsstufe parallel geschaltet, und die Anlage umfasst ferner eine Leitung zum Einleiten eines Teils des an Methanol abgereicherten Spülgasstroms in die Membrantrennstufe und eine Leitung zum Einleiten des verbliebenen Teils des an Methanol abgereicherten Spülgasstroms in die Druckwechseladsorptionsstufe. Wie bereits oben erläutert, empfiehlt sich diese Ausgestaltung, wenn ein Teil des mittels PSA abgetrennten, hochreinen Wasserstoffs an Verbraucher außerhalb des Verfahrens exportiert werden soll.

In Weiterbildung der zuvor erörterten Ausgestaltung der erfindungsgemäßen Anlage umfasst diese ferner eine Leitung, mit der ein erster an Wasserstoff angereicherter Gasstrom, der in der Membrantrennstufe als Permeat gewonnen wird, als Trimmgasstrom zu der ersten Mischvorrichtung zurückgeführt wird. Die relativ geringe Wasserstoffreinheit im Permeat reicht oft bereits für eine Einstellung der erwünschten Stöchiometriezahl im Synthesegas-Feedgasstrom aus, insbesondere dann, wenn das Wasserstoffdefizit nur gering ist.

In weiterer Weiterbildung der zuvor erörterten Ausgestaltung der erfindungsgemäßen Anlage umfasst diese auch eine Leitung zum Einleiten eines zweiten an Wasserstoff angereicherten Gasstroms, der in der Druckwechseladsorptionsstufe gewonnen wird, als Trimmgasstrom zu der ersten Mischvorrichtung oder zum Ausleiten aus der Anlage als Wasserstoff-Exportstrom. Eine Verwendung als weiterer Trimmgasstrom bringt vor allem dann Vorteile, wenn der Synthesegas-Feedgasstrom ein größeres Wasserstoffdefizit aufweist. Ansonsten kann in der in der Druckwechseladsorptionsstufe erhaltene, an Wasserstoff angereicherte Gasstrom als Wasserstoff-Exportstrom an externe Verbraucher abgegeben werden.

## Ausführungs- und Zahlenbeispiele

Weitere Merkmale, Vorteile und Anwendungsmöglichkeiten der Erfindung ergeben sich auch aus der nachfolgenden Beschreibung von Ausführungs- und Zahlenbeispielen und der Zeichnungen. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

Es zeigen:

Fig. 1    die schematische Darstellung des erfindungsgemäßen Verfahrens bzw. der erfindungsgemäßen Anlage nach einer ersten Ausgestaltung,

Fig. 2    die schematische Darstellung des erfindungsgemäßen Verfahrens bzw. der erfindungsgemäßen Anlage nach einer zweiten Ausgestaltung,

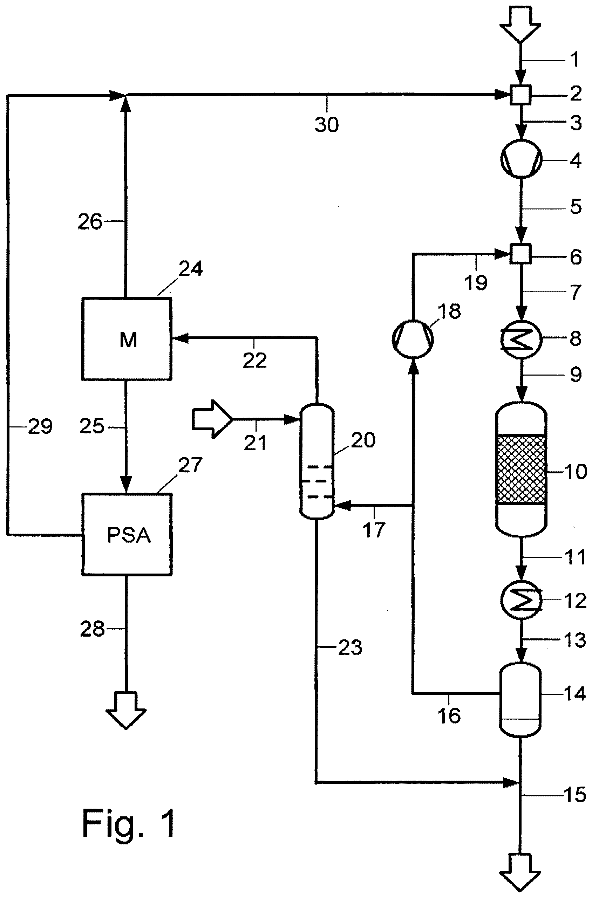

In Fig. 1 wird das erfindungsgemäße Verfahren bzw. die erfindungsgemäße Anlage nach einer ersten Ausgestaltung erläutert, deren Ziel die maximale Wiedergewinnung von Wasserstoff aus dem Purgegas der Methanolsynthese ist.

Über die Leitung 1 wird ein Wasserstoff und Kohlenstoffoxide enthaltender Synthesegas-Frischgasstrom, der bezüglich der für die Methanolsynthese vorgegebenen Stöchiometrie ein Wasserstoffdefizit enthält, zu einer ersten Mischvorrichtung 2 geführt. In der ersten Mischvorrichtung 2 erfolgt ein Zusammenführen des an Wasserstoff defizitären Synthesegas-Frischgasstroms mit einem Wasserstoff enthaltenden Trimmgasstrom, der über Leitung 30 zu der ersten Mischvorrichtung geführt wird, zu einem an Wasserstoff angereicherten Synthesegas-Frischgasstrom. Dieser wird über Leitung 3 zum Verdichter 4 geführt und dort auf den Synthesedruck der Methanolsynthese verdichtet.

Über Leitung 5 wird der verdichtete, an Wasserstoff angereicherte Synthesegas-Frischgasstrom zu der zweiten Mischvorrichtung 6 geführt und dort mit dem über Leitung 19 herangeführten Synthesegas-Kreislaufgasstrom zu einem Synthesegas-Feedgasstrom vereinigt. Über Leitung 7 wird der Synthesegas-Feedgasstrom zu der Heizvorrichtung 8 geführt und dort auf die Reaktionstemperatur der Methanolsynthese erhitzt. Die Heizvorrichtung 8 umfasst bevorzugt einen Wärmetauscher, mit dem die bei der Abkühlung des Produktstroms des Methanolsynthesereaktors abgeführte Wärmeenergie mindestens teilweise auf den in den Reaktor eintretenden Synthesegas-Feedgasstrom übertragen wird. Der Synthesegas-Feedgasstrom wird auf eine Temperatur zwischen 180 und 280 °C, bevorzugt zwischen 190 und 250 °C aufgeheizt.

Über Leitung 9 wird der auf die Reaktionstemperatur der Methanolsynthese erhitzte Synthesegas-Feedgasstrom in den Methanolsynthesereaktor 10 eingeleitet. In diesem erfolgt ein teilweises, katalytisches Umsetzen der in dem Synthesegas-Feedgasstrom enthaltenen Kohlenstoffoxide mit Wasserstoff unter Methanolsynthesebedingungen zu Methanol. Der Methanolsynthesereaktor kann dabei aus einer Verschaltung mehrerer Einzelreaktoren bestehen, die beispielsweise gasgekühlt bzw. wassergekühlt sind, wie es die Patentschrift EP 0 790 226 B1 lehrt.

Der Produktstrom, der im Methanolsynthesereaktor gebildetes Methanol sowie nicht umgesetzte Synthesegasbestandteile umfasst, verlässt über Leitung 11 den Methanolsynthesereaktor, wird im Kühler 12 abgekühlt und mittels Leitung 13 zu der Phasentrennvorrichtung 14 geführt. Wie bereits oben angedeutet, kann der Kühler einen Wärmetauscher umfassen, wobei die abzuführende Wärmeenergie im indirekten Wärmetausch auf den kalten Synthesegas-Feedgasstrom übertragen und letzterer somit aufgeheizt wird.

In der Phasentrennvorrichtung 14 wird der abgekühlte Produktstrom des Methanolsynthesereaktors in eine flüssige Phase sowie in eine Gasphase aufgetrennt. Die gewonnene flüssige Phase enthält erzeugtes Methanol und Wasser als Koppelprodukt; sie wird über Leitung 15 zu einer bildlich nicht dargestellten Destillationsvorrichtung geführt, in der Methanol und Wasser getrennt werden und somit Reinmethanol gewonnen wird. Die in der Phasentrennvorrichtung 14 erhaltene Gasphase enthält nicht umgesetzte Synthesegasbestandteile sowie gegebenenfalls Inertgasbestandteile. Sie wird über Leitung 16, Kreislaufverdichter 18 und Leitung 19 als Synthesegas-Kreislaufgasstrom zu der zweiten Mischvorrichtung 6 zurückgeführt.

Über Leitung 17 wird ein Teil des Synthesegas-Kreislaufgasstroms als Spülgasstrom (Purgegas) aus dem Synthesegas-Kreislauf abgeführt und zu der Waschvorrichtung 20 geleitet. In der Waschvorrichtung wird der Spülgasstrom mit demineralisiertem Wasser als Waschmittel beaufschlagt, das über Leitung 21 herangeführt wird. Es können aber auch andere, insbesondere Wasserqualitäten höherer Reinheit, beispielsweise Reinstwasser oder destilliertes Wasser, als Waschmittel eingesetzt werden. Wasser geringerer Reinheit ist dann als Waschmittel einsetzbar, wenn die vorhandenen Begleitstoffe in nachgeschalteten Verfahrensstufen keine Probleme bereiten, Über Leitung 22 erfolgt das Ausleiten eines hinsichtlich seiner Methanolbeladung reduzierten Spülgasstroms aus dem oberen Bereich der Waschkolonne, während über Leitung 23 das mit Methanol beladene Waschmittel aus dem unteren Bereich der Waschkolonne abgeführt wird. Das mit Methanol beladene Waschmittel wird mit der in Leitung 15 abgeführten flüssigen Phase aus der Phasentrennvorrichtung vereinigt und zu der bildlich nicht dargestellten Destillationsvorrichtung geführt.

Der hinsichtlich seiner Methanolbeladung reduzierte Spülgasstrom wird mittels eines bildlich nicht dargestellten Wärmetauschers überhitzt, um Kondensation vor bzw. in der Membrantrennstufe zu verhindern, und über Leitung 22 zu der Membrantrennstufe 24 geführt. Er wird dort in einen an Wasserstoff angereicherten Permeatstrom und einen an Wasserstoff abgereicherter Retentatstrom aufgetrennt. Der an Wasserstoff angereicherte Permeatstrom wird als Wasserstoff enthaltender Trimmgasstrom über Leitungen 26 und 30 zu der ersten Mischvorrichtung 2 zurückgeführt und dient somit zur Einstellung der gewünschten Stöchiometriezahl R. Die Einstellung erfolgt dabei über eine im Leitungsweg der Leitungen 26 und 30 angeordnete, bildlich nicht dargestellte Mengenstromregelung. Falls das Wasserstoffdefizit des Synthesegas-Frischgasstroms nur klein ist und nicht der gesamte Permeatstrom zur Einstellung der gewünschten Stöchiometriezahl benötigt wird, kann der überschüssige Anteil über eine bildlich nicht dargestellte Leitung aus der Membrantrennstufe abgeführt und beispielsweise als Heizgas genutzt werden. Er kann auch an externe Verbraucher abgegeben werden, die einen Gasstrom mit relativ geringer Wasserstoffreinheit verarbeiten können.

Der an Wasserstoff abgereicherte Retentatstrom wird über Leitung 25 aus der Membrantrennstufe abgeführt und der Druckwechseladsorptionsstufe 27 aufgegeben. In der Druckwechseladsorptionsstufe wird ein zweiter an Wasserstoff angereicherter Gasstrom gewonnen, der über Leitung 29 abgeführt und gemeinsam mit dem in Leitungen 26 und 30 geführten Permeatstrom aus der Membrananlage als Trimmgasstrom mit dem an Wasserstoff defizitären Synthesegas-Frischgasstrom zusammengeführt wird. Wenn, wie oben dargelegt, nicht der gesamte, an Wasserstoff angereicherte Gasstrom aus der Druckwechseladsorptionsstufe zur Einstellung der gewünschten Stöchiometriezahl benötigt wird, kann der überschüssige Anteil über eine bildlich nicht dargestellte Leitung als Wasserstoff-Exportstrom aus dem Verfahren ausgeleitet und an externe Verbraucher abgegeben werden.

Über Leitung 28 wird der an Wasserstoff verarmte Restgasstrom (PSA-Tailgas) aus der Druckwechseladsorptionsstufe abgeführt. Er enthält noch brennbare Komponenten, beispielsweise Kohlenmonoxid, und kann daher beispielsweise als Heizgas genutzt werden. Auch eine stoffliche Nutzung des Restgasstroms, beispielsweise in einer benachbarten Anlage zur Kohlevergasung, ist möglich.

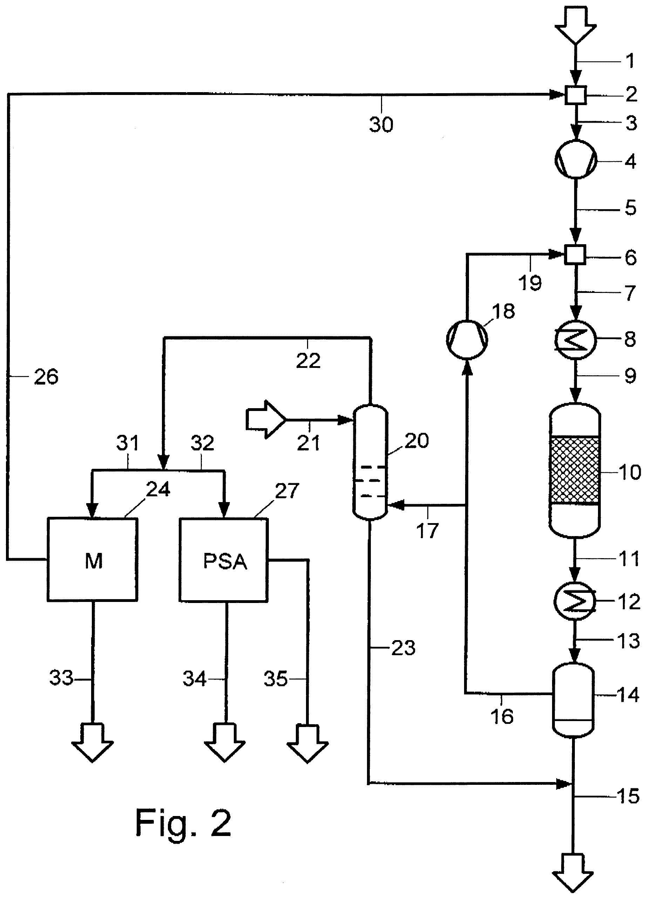

In Fig. 2 entspricht das erfindungsgemäße Verfahren bzw. die erfindungsgemäße Anlage bis zum Bezugszeichen 23 der oben erörterten ersten Ausgestaltung. Im Unterschied zu letzterer wird aber nunmehr in der zweiten Ausgestaltung der Erfindung der über Leitung 22 herangeführte, an Methanol abgereicherte Spülgasstrom mittels der Leitungen 31 und 32 parallel zu der der Membrantrennstufe 24 und der Druckwechseladsorptionsstufe 27 geführt und diesen aufgegeben. Die Verteilung der zu der Membrantrennstufe 24 und der Druckwechseladsorptionsstufe 27 geführten Teilgasströme ist nicht notwendigerweise gleich; sie wird sich vielmehr über die zur Einstellung der Stöchiometriezahl benötigten Menge des in der Membrantrennstufe 24 gewonnenen und über Leitungen 26 und 30 geführten, an Wasserstoff angereicherten Permeatstroms richten. Eine Gleichverteilung der beiden Ströme ist aber durchaus möglich und beispielsweise dann erwünscht, wenn ein entsprechend großer Wasserstoff-Exportstrom aus der Druckwechseladsorptionsstufe an externe Verbraucher abgegeben werden soll.

Die Verteilung auf die beiden Trennstufen wird dabei über im Leitungsweg der Leitungen 31 und 32 angeordnete, bildlich nicht dargestellte Mengenstromregelungen vorgenommen. Über die Leitungen 26 und 30 wird der an Wasserstoff angereicherte Permeatstrom aus der Membrantrennstufe als Trimmgasstrom zu der ersten Mischvorrichtung geführt und dort mit dem an Wasserstoff defizitären Synthesegas-Frischgasstrom zusammengeführt.

Über Leitung 35 wird ein Reinwasserstoffstrom mit einer Wasserstoffreinheit von typischerweise über 99 Vol.-% aus der Druckwechseladsorptionsstufe 27 abgeführt und als Exportstrom an externe Verbraucher abgegeben.

Über die Leitungen 33 und 34 werden der an Wasserstoff abgereicherte Retentatstrom aus der Membrantrennstufe und der ebenfalls an Wasserstoff abgereicherte Restgasstrom (PSA-Tailgas) aus der Druckwechseladsorptionsstufe abgeführt. Beide enthalten noch brennbare Bestandteile, beispielsweise Kohlenmonoxid, und können somit hinsichtlich ihres Heizwertes thermisch genutzt werden, beispielsweise in einer der Methanolsynthese vorgeschalteten Reformeranlage zur Unterfeuerung des Reformerofens. Auch eine stoffliche Nutzung des Retentatstroms und des Restgasstroms, beispielsweise in einer benachbarten Anlage zur Kohlevergasung, ist möglich.

Zahlenbeispiele

In den nachfolgenden Zahlenbeispielen wird die Abtrennung von Methanol aus dem mit Methanolresten beladenen Spülgasstrom (Purgegas) in der Waschvorrichtung dargestellt (Tabelle 1). Ferner werden die Abtrennung und Rückführung des Wasserstoffs bei Serienschaltung (Tabelle 2) und bei Parallelschaltung (Tabelle 3) der Membrantrennstufe und der Druckwechseladsorptionsstufe demonstriert, wie sie in den obigen Ausführungsbeispielen anhand Fig. 1 und Fig. 2 erläutert wurden.

Die in den Tabellen 2 und 3 wiedergegebenen Daten verdeutlichen die Wirkungsweisen und Anwendungsfälle der beiden beschriebenen Ausgestaltungen der Erfindung. So können bei Serienschaltung von Membrantrennstufe und Druckwechseladsorptionsstufe laut Fig. 1 und Tabelle 2 insgesamt 967 kmol/h Wasserstoff zu der Methanolsynthese zurückgeführt werden.

Bei Parallelschaltung laut Fig. 2 und Tabelle 3 werden insgesamt nur 361 kmol/h Wasserstoff zu der Methanolsynthese zurückgeführt, jedoch werden weitere 412 kmol/h hochreinen Wasserstoffs als Exportstrom gewonnen.

**Tabelle 1:** Abtrennung von Methanol aus dem mit Methanolresten beladenen Spülgasstrom (Purgegas) in der Waschvorrichtung

| | Fig. 1 und 2: Purgegaswäsche | | | | |
|---|---|---|---|---|---|
| | | | | | |
| Leitung | 17 | 21 | 23 | 22 | 22-WT[1)] |
| | | | | | |
| Molenbrüche: | | | | | |
| Methanol | 0,006 | 0,000 | 0,046 | 0,000 | 0,000 |
| $H_2O$ | 0,000 | 1,000 | 0,952 | 0,001 | 0,001 |
| $CO_2$ | 0,042 | 0,000 | 0,001 | 0,042 | 0,042 |
| CO | 0,064 | 0,000 | 0,000 | 0,065 | 0,065 |
| $H_2$ | 0,799 | 0,000 | 0,001 | 0,803 | 0,803 |
| Ar | 0,000 | 0,000 | 0,000 | 0,000 | 0,000 |
| $N_2$ | 0,088 | 0,000 | 0,000 | 0,089 | 0,089 |
| $CH_4$ | 0,000 | 0,000 | 0,000 | 0,000 | 0,000 |
| Gesamtstrom kmol/h | 1288 | 153 | 159 | 1282 | 1282 |

(fortgesetzt)

| | Fig. 1 und 2: Purgegaswäsche | | | | |
|---|---|---|---|---|---|
| | | | | | |
| **Leitung** | **17** | **21** | **23** | **22** | **22-WT**[1] |
| | | | | | |
| **Molenbrüche:** | | | | | |
| Gesamtstrom kg/h | 10224 | 2755 | 2968 | 10010 | 10010 |
| Gesamtstrom m$^3$/h | 490 | 3 | 3 | 498 | 508 |
| Temperatur °C | 40 | 42 | 45 | 44 | 50 |
| Druck MPa,g | 7,0 | 8,1 | 7,0 | 7,0 | 7,0 |
| [1] Strom in Leitung 22 nach Erhitzen in Wärmetauscher, bildlich nicht dargestellt | | | | | |

**Tabelle 2**: Abtrennung und Rückführung des Wasserstoffs bei Serienschaltung der Membrantrennstufe und der Druckwechseladsorptionsstufe

| | Fig. 1: Serienschaltung Membran + PSA | | | | |
|---|---|---|---|---|---|
| | | | | | |
| **Leitung** | **26** | **25** | **28** | **29** | **30** |
| | | | | | |
| **Molenbrüche:** | | | | | |
| Methanol | 0,000 | 0,000 | 0,000 | 0,000 | 0,000 |
| $H_2O$ | 0,002 | 0,001 | 0,002 | 0,000 | 0,001 |
| $CO_2$ | 0,042 | 0,044 | 0,092 | 0,000 | 0,032 |
| CO | 0,034 | 0,118 | 0,248 | 0,000 | 0,026 |
| $H_2$ | 0,888 | 0,656 | 0,276 | 0,999 | 0,914 |
| Ar | 0,000 | 0,000 | 0,000 | 0,000 | 0,000 |
| $N_2$ | 0,035 | 0,181 | 0,381 | 0,000 | 0,027 |
| $CH_4$ | 0,000 | 0,000 | 0,001 | 0,000 | 0,000 |
| Gesamtstrom kmol/h | 811 | 471 | 224 | 247 | 1058 |
| Gesamtstrom kg/h | 4524 | 5486 | 4983 | 503 | 5027 |
| Gesamtstrom m$^3$/h | 672 | 186 | 2986 | 130 | 905 |
| Temperatur °C | 50 | 50 | 50 | 50 | 50 |
| Druck MPa,g | 3,2 | 7,0 | 0,1 | 5,2 | 3,1 |

**Tabelle 3**: Abtrennung und Rückführung des Wasserstoffs bei Parallelschaltung der Membrantrennstufe und der Druckwechseladsorptionsstufe

| Fig. 2: Parallelschaltung Membran + PSA | | | | | | |
|---|---|---|---|---|---|---|
| | | | | | | |
| Leitung | 31 | 26 | 33 | 32 | 34 | 35 |
| | | | | | | |
| **Molenbrüche:** | | | | | | |
| Methanol | 0,000 | 0,000 | 0,000 | 0,000 | 0,000 | 0,000 |
| $H_2O$ | 0,001 | 0,002 | 0,001 | 0,001 | 0,004 | 0,000 |
| $CO_2$ | 0,042 | 0,042 | 0,044 | 0,042 | 0,119 | 0,000 |
| CO | 0,065 | 0,034 | 0,118 | 0,065 | 0,180 | 0,000 |
| $H_2$ | 0,803 | 0,888 | 0,656 | 0,803 | 0,449 | 1,000 |
| Ar | 0,000 | 0,000 | 0,000 | 0,000 | 0,000 | 0,000 |
| $N_2$ | 0,089 | 0,035 | 0,181 | 0,089 | 0,248 | 0,000 |
| $CH_4$ | 0,000 | 0,000 | 0,000 | 0,000 | 0,001 | 0,000 |
| G esamtst rom kmol/h | 641 | 406 | 235 | 641 | 229 | 412 |
| Gesamtstrom kg/h | 5005 | 2262 | 2743 | 5005 | 4171 | 834 |
| Gesamtstrom $m^3$/h | 254 | 161 | 93 | 259 | 3062 | 223 |
| Temperatur °C | 50 | 50 | 50 | 50,0 | 50,0 | 50,2 |
| Druck MPa,g | 7,0 | 7,0 | 7,0 | 6,8 | 0,1 | 5,0 |

**Gewerbliche Anwendbarkeit**

Mit der Erfindung werden eine Anlage und ein Verfahren zur Verarbeitung von $H_2$-defizitärem Synthesegas in der Methanolsynthese zur Verfügung gestellt. Gegenüber den aus dem Stand der Technik bekannten Verfahren bietet die Erfindung den Vorteil, dass der zum Ausgleich des Defizits benötigte Wasserstoff aus dem Purgegas der Methanolsynthese gewonnen wird und nicht etwa aus dem Synthesegas-Frischgasstrom. Es fällt daher kein CO und $CO_2$ enthaltender Abfallstrom stromaufwärts der Methanolsynthese an, so dass alle im Frischgasstrom enthaltenen Kohlenoxide in die Methanolsynthese gelangen.

Besonders günstig ist es, wenn die Erfindung durch Vorsehen entsprechender zusätzlicher Leitungen und Absperrorgane so ausgestaltet wird, dass sich die beiden in Fig. 1 und 2 dargestellten Ausgestaltungen durch einfaches Umschalten ineinander überführen lassen. Hierdurch kann flexibel auf das Wasserstoffdefizit des verwendeten Synthesegases sowie auf einen geänderten Wasserstoffbedarf externer Verbraucher reagiert werden.

**Bezugszeichenliste**

| | |
|---|---|
| 1 | Leitung |
| 2 | erste Mischvorrichtung |
| 3 | Leitung |
| 4 | Verdichter |
| 5 | Leitung |
| 6 | zweite Mischvorrichtung |
| 7 | Leitung |
| 8 | Heizvorrichtung |
| 9 | Leitung |
| 10 | Methanolsynthesereaktor |

| 11 | Leitung |
| 12 | Kühler |
| 13 | Leitung |
| 14 | Phasentrennvorrichtung |
| 15 - 17 | Leitung |
| 18 | Kreislaufverdichter |
| 19 | Leitung |
| 20 | Waschvorrichtung |
| 21 - 23 | Leitung |
| 24 | Membrantrennstufe (M) |
| 25 - 26 | Leitung |
| 27 | Druckwechseladsorptionsstufe (PSA) |
| 28 - 35 | Leitung |

**Patentansprüche**

1. Verfahren zur Herstellung von Methanol aus einem Wasserstoff, Kohlenstoffoxide und optional Inertkomponenten enthaltenden Synthesegas, umfassend folgende Schritte:

    (a) Bereitstellen eines Wasserstoff, Kohlenstoffoxide und optional Inertkomponenten enthaltenden Synthesegas-Frischgasstroms, der bezüglich der für die Methanolsynthese vorgegebenen Stöchiometrie ein Wasserstoffdefizit enthält,

    (b) Zusammenführen des an Wasserstoff defizitären Synthesegas-Frischgasstroms mit einem Wasserstoff enthaltenden Trimmgasstrom zu einem an Wasserstoff angereicherten Synthesegas-Frischgasstrom,

    (c) Zusammenführen des an Wasserstoff angereicherten Synthesegas-Frischgasstroms mit dem in Schritt (g) erhaltenen Synthesegas-Kreislaufgasstrom zu einem Synthesegas-Feedgasstrom,

    (d) Einleiten des Synthesegas-Feedgasstroms in mindestens einen Methanolsynthesereaktor und mindestens teilweises, katalytisches Umsetzen der in dem Synthesegas-Feedgasstrom enthaltenen Kohlenstoffoxide mit Wässerstoff unter Methanolsynthesebedingungen zu Methanol,

    (e) Ausleiten eines Methanoldampf und nicht umgesetzte Synthesegasbestandteile enthaltenden Produktgasstroms aus dem mindestens einen Methanolsynthesereaktor,

    (f) Mindestens teilweises Abtrennen des Methanols aus dem Produktgasstrom durch Abkühlen und Kondensation,

    (g) Auftrennen des Produktgasstroms nach Abtrennen des Methanols in einen Synthesegas-Kreislaufgasstrom und einen mit Methanolresten beladenen Spülgasstrom, Rückführen des Synthesegas-Kreislaufgasstroms nach Schritt (c),

    (h) Einleiten des mit Methanolresten beladenen Spülgasstroms in eine Waschvorrichtung, Inkontaktbringen des mit Methanolresten beladenen Spülgasstroms in der Waschvorrichtung mit einem Waschmittel, bevorzugt Wasser, Ausleiten eines an Methanol abgereicherten Spülgasstroms und eines mit Methanol beladenen Waschmittels aus der Waschvorrichtung,

    (i) Einleiten des an Methanol abgereicherten Spülgasstroms in eine Wasserstoffabtrennungsvorrichtung, die eine Membrantrennstufe und eine Druckwechseladsorptionsstufe umfasst, wobei mindestens ein an Wasserstoff angereicherter und mindestens ein an Wasserstoff abgereicherter Gasstrom erhalten wird,

    (j) Rückführen mindestens eines an Wasserstoff angereicherten Gasstroms als Wasserstoff enthaltender Trimmgasstrom nach Schritt (b).

2. Verfahren nach Anspruch 1, **dadurch gekennzeichnet, dass** in der Wasserstoffabtrennungsvorrichtung die Membrantrennstufe und die Druckwechseladsorptionsstufe hintereinander geschaltet sind und in Fluidverbindung miteinander stehen.

3. Verfahren nach Anspruch 2, **dadurch gekennzeichnet, dass** in der Wasserstoffabtrennungsvorrichtung zunächst die Membrantrennstufe und anschließend die Druckwechseladsorptionsstufe von dem an Methanol abgereicherten Spülgasstrom durchströmt werden.

4. Verfahren nach Anspruch 3, **dadurch gekennzeichnet, dass** in der Membrantrennstufe als Permeat ein erster an Wasserstoff angereicherter Gasstrom gewonnen wird, der als Trimmgasstrom mit dem an Wasserstoff defizitären Synthesegas-Frischgasstrom zusammengeführt wird.

5. Verfahren nach Anspruch 4, **dadurch gekennzeichnet, dass** das in der Membrantrennstufe erhaltene Retentat der Druckwechseladsorptionsstufe aufgegeben und in der Druckwechseladsorptionsstufe ein zweiter an Wasserstoff angereicherter Gasstrom gewonnen wird, der als Trimmgasstrom mit dem an Wasserstoff defizitären Synthesegas-Frischgasstrom zusammengeführt wird oder als Wasserstoff-Exportstrom aus dem Verfahren ausgeleitet wird.

6. Verfahren nach Anspruch 5, **dadurch gekennzeichnet, dass** der zweite an Wasserstoff angereicherte Gasstrom zuerst mit dem ersten an Wasserstoff angereicherten Gasstrom und anschließend als Trimmgasstrom mit dem an Wasserstoff defizitären Synthesegas-Frischgasstrom zusammengeführt wird.

7. Verfahren nach Anspruch 1, **dadurch gekennzeichnet, dass** in der Wasserstoffabtrennungsvorrichtung die Membrantrennstufe und die Druckwechseladsorptionsstufe parallel geschaltet sind, wobei ein Teil des an Methanol abgereicherten Spülgasstroms der Membrantrennstufe und der verbliebene Teil des an Methanol abgereicherten Spülgasstroms der Druckwechseladsorptionsstufe aufgegeben wird.

8. Verfahren nach Anspruch 7, **dadurch gekennzeichnet, dass** in der Membrantrennstufe als Permeat ein erster an Wasserstoff angereicherter Gasstrom gewonnen wird, der als Trimmgasstrom mit dem an Wasserstoff defizitären Synthesegas-Frischgasstrom zusammengeführt wird.

9. Verfahren nach Anspruch 7, **dadurch gekennzeichnet, dass** in der Druckwechseladsorptionsstufe ein zweiter an Wasserstoff angereicherter Gasstrom gewonnen wird, der als Trimmgasstrom mit dem an Wasserstoff defizitären Synthesegas-Frischgasstrom zusammengeführt wird oder als Wasserstoff-Exportstrom aus dem Verfahren ausgeleitet wird.

10. Verfahren nach einem der vorigen Ansprüche, **dadurch gekennzeichnet, dass** das Bereitstellen des Synthesegas-Frischgasstroms mittels einer Synthesegaserzeugungsstufe erfolgt, die einen Autothermreformer umfasst.

11. Anlage zur Herstellung von Methanol aus einem Wasserstoff, Kohlenstoffoxide und optional Inertkomponenten enthaltenden Synthesegas, umfassend folgende Anlagenkomponenten:

    (a) eine Synthesegaserzeugungsstufe zum Bereitstellen eines Wasserstoff, Kohlenstoffoxide und optional Inertkomponenten enthaltenden Synthesegas-Frischgasstroms, der bezüglich der für die Methanolsynthese vorgegebenen Stöchiometrie ein Wasserstoffdefizit enthält,
    (b) eine erste Mischvorrichtung zum Zusammenführen des an Wasserstoff defizitären Synthesegas-Frischgasstroms mit einem Wasserstoff enthaltenden Trimmgasstrom zu einem an Wasserstoff angereicherten Synthesegas-Frischgasstrom,
    (c) eine zweite Mischvorrichtung zum Zusammenführen des an Wasserstoff angereicherten Synthesegas-Frischgasstroms mit einem Synthesegas-Kreislaufgasstrom zu einem Synthesegas-Feedgasstrom,
    (d) mindestens einen Methanolsynthesereaktor, eine Leitung zum Einleiten des Synthesegas-Feedgasstroms in den mindestens einen Methanolsynthesereaktor,
    (e) eine Leitung zum Ausleiten eines Methanoldampf und nicht umgesetzte Synthesegasbestandteile enthaltenden Produktgasstroms aus dem mindestens einen Methanolsynthesereaktor,
    (f) eine Abtrennvorrichtung zum mindestens teilweisen Abtrennen des Methanols aus dem Produktgasstrom durch Abkühlen und Kondensation,
    (g) eine Auftrennvorrichtung zum Auftrennen des Produktgasstroms nach Abtrennen des Methanols in einen Synthesegas-Kreislaufgasstrom und einen mit Methanolresten beladenen Spülgasstrom, sowie eine Leitung zum Rückführen des Synthesegas-Kreislaufgasstroms zu der zweiten Mischvorrichtung,
    (h) eine Waschvorrichtung, eine Leitung zum Einleiten des mit Methanolresten beladenen Spülgasstroms in die Waschvorrichtung, eine Leitung zum Einleiten des Waschmittels, eine Leitung zum Ausleiten eines an Methanol abgereicherten Spülgasstroms aus der Waschvorrichtung und eine Leitung zum Ausleiten eines mit Methanol beladenen Waschmittels aus der Waschvorrichtung,
    (i) eine Wasserstoffabtrennungsvorrichtung, umfassend eine Membrantrennstufe und eine Druckwechseladsorptionsstufe, eine Leitung zum Einleiten des an Methanol abgereicherten Spülgasstroms in die Wasserstoffabtrennungsvorrichtung, eine Leitung zum Ausleiten eines an Wasserstoff angereicherten Gasstroms aus der Wasserstoffabtrennungsvorrichtung und eine Leitung zum Ausleiten eines an Wasserstoff abgereicherten Gasstroms aus der Wasserstoffabtrennungsvorrichtung,
    (j) eine Leitung zum Rückführen eines an Wasserstoff angereicherten Gasstroms als Wasserstoff enthaltender Trimmgasstrom zu der ersten Mischvorrichtung.

**12.** Anlage nach Anspruch 11, **dadurch gekennzeichnet, dass** in der Wasserstoffabtrennungsvorrichtung die Membrantrennstufe und die Druckwechseladsorptionsstufe hintereinander geschaltet sind und in Fluidverbindung miteinander stehen.

**13.** Anlage nach Anspruch 12, **dadurch gekennzeichnet, dass** in der Wasserstoffabtrennungsvorrichtung zunächst die Membrantrennstufe und anschließend die Druckwechseladsorptionsstufe von dem an Methanol abgereicherten Spülgasstrom durchströmt werden.

**14.** Anlage nach Anspruch 13, ferner umfassend eine Leitung, mit der ein erster an Wasserstoff angereicherter Gasstrom, der in der Membrantrennstufe als Permeat gewonnen wird, als Trimmgasstrom zu der ersten Mischvorrichtung zurückgeführt wird.

**15.** Anlage nach Anspruch 14, ferner umfassend eine Leitung zum Einleiten des in der Membrantrennstufe erhaltenen Retentats in die Druckwechseladsorptionsstufe und eine Leitung zum Einleiten eines zweiten an Wasserstoff angereicherten Gasstroms, der in der Druckwechseladsorptionsstufe gewonnen wird, als Trimmgasstrom in die erste Mischvorrichtung oder zum Ausleiten aus der Anlage als Wasserstoff-Exportstrom.

**16.** Anlage nach Anspruch 15, ferner umfassend eine Mischvorrichtung zum Zusammenführen des zweiten an Wasserstoff angereicherten Gasstroms mit dem ersten an Wasserstoff angereicherter Gasstrom, sowie eine Leitung zum Zuführen des dabei erhaltenen Mischgasstroms zu der ersten Mischvorrichtung.

**17.** Anlage nach Anspruch 11, **dadurch gekennzeichnet, dass** in der Wasserstoffabtrennungsvorrichtung die Membrantrennstufe und die Druckwechseladsorptionsstufe parallel geschaltet sind, und ferner umfassend eine Leitung zum Einleiten eines Teils des an Methanol abgereicherten Spülgasstroms in die Membrantrennstufe und eine Leitung zum Einleiten des verbliebenen Teils des an Methanol abgereicherten Spülgasstroms in die Druckwechseladsorptionsstufe.

**18.** Anlage nach Anspruch 17, ferner umfassend eine Leitung, mit der ein erster an Wasserstoff angereicherter Gasstrom, der in der Membrantrennstufe als Permeat gewonnen wird, als Trimmgasstrom zu der ersten Mischvorrichtung zurückgeführt wird.

**19.** Anlage nach Anspruch 18, ferner umfassend eine Leitung zum Einleiten eines zweiten an Wasserstoff angereicherten Gasstroms, der in der Druckwechseladsorptionsstufe gewonnen wird, als Trimmgasstrom zu der ersten Mischvorrichtung oder zum Ausleiten aus der Anlage als Wasserstoff-Exportstrom.

**Claims**

**1.** A process for producing methanol from a synthesis gas containing hydrogen, carbon oxides and optionally inert components, comprising the following steps:

(a) providing a synthesis gas make-up gas stream containing hydrogen, carbon oxides and optionally inert components, which contains a hydrogen deficit with respect to the stoichiometry specified for the methanol synthesis,

(b) combining the synthesis gas make-up gas stream deficient in hydrogen with a trimming gas stream containing hydrogen to obtain a synthesis gas make-up gas stream enriched in hydrogen,

(c) combining the synthesis gas make-up gas stream enriched in hydrogen with the synthesis gas cycle gas stream obtained in step (g) to obtain a synthesis gas feed gas stream,

(d) introducing the synthesis gas feed gas stream into at least one methanol synthesis reactor and at least in part catalytically converting the carbon oxides contained in the synthesis gas feed gas stream to methanol by using hydrogen under methanol synthesis conditions,

(e) discharging a product gas stream containing methanol vapor and non-converted synthesis gas constituents from the at least one methanol synthesis reactor,

(f) at least partly separating the methanol from the product gas stream by cooling and condensation,

(g) fractionating the product gas stream after separation of the methanol into a synthesis gas cycle gas stream and a purge gas stream loaded with methanol residues, recirculating the synthesis gas cycle gas stream to step (c),

(h) introducing the purge gas stream loaded with methanol residues into a washing device, contacting the purge gas stream loaded with methanol residues in the washing device with a washing agent, preferably water, discharging a purge gas stream depleted of methanol and a washing agent loaded with methanol from the washing device,

(i) introducing the purge gas stream depleted of methanol into a hydrogen separation device which comprises a membrane separation stage and a pressure swing adsorption stage, wherein at least one gas stream enriched in hydrogen and at least one gas stream depleted of hydrogen is obtained,

(j) recirculating at least one gas stream enriched in hydrogen as trimming gas stream containing hydrogen to step (b).

2. The process according to claim 1, **characterized in that** in the hydrogen separation device the membrane separation stage and the pressure swing adsorption stage are connected in series and are in fluid connection with each other.

3. The process according to claim 2, **characterized in that** in the hydrogen separation device first the membrane separation stage and then the pressure swing adsorption stage are traversed by the purge gas stream depleted of methanol.

4. The process according to claim 3, **characterized in that** a first gas stream enriched in hydrogen is obtained as permeate in the membrane separation stage, which as trimming gas stream is combined with the synthesis gas make-up gas stream deficient in hydrogen.

5. The process according to claim 4, **characterized in that** the retentate obtained in the membrane separation stage is charged to the pressure swing adsorption stage, and in the pressure swing adsorption stage a second gas stream enriched in hydrogen is obtained, which as trimming gas stream is combined with the synthesis gas make-up gas stream deficient in hydrogen or is discharged from the process as hydrogen export stream.

6. The process according to claim 5, **characterized in that** the second gas stream enriched in hydrogen is combined first with the first gas stream enriched in hydrogen and subsequently as trimming gas stream with the synthesis gas make-up gas stream deficient in hydrogen.

7. The process according to claim 1, **characterized in that** the membrane separation stage and the pressure swing adsorption stage are connected in parallel in the hydrogen separation device, wherein a part of the purge gas stream depleted of methanol is charged to the membrane separation stage and the remaining part of the purge gas stream depleted of methanol is charged to the pressure swing adsorption stage.

8. The process according to claim 7, **characterized in that** a first gas stream enriched in hydrogen is obtained as permeate in the membrane separation stage, which as trimming gas stream is combined with the synthesis gas make-up gas stream deficient in hydrogen.

9. The process according to claim 7, **characterized in that** a second gas stream enriched in hydrogen is obtained in the pressure swing adsorption stage, which as trimming gas stream is combined with the synthesis gas make-up gas stream deficient in hydrogen or is discharged from the process as hydrogen export stream.

10. The process according to any of the preceding claims, **characterized in that** the provision of the synthesis gas make-up gas stream is effected by means of a synthesis gas generation stage which comprises an autothermal reformer.

11. A plant for producing methanol from a synthesis gas containing hydrogen, carbon oxides and optionally inert components, comprising the following plant components:

(a) a synthesis gas generation stage for providing a synthesis gas make-up gas stream containing hydrogen, carbon oxides and optionally inert components, which contains a hydrogen deficit with respect to the stoichiometry specified for the methanol synthesis,

(b) a first mixing device for combining the synthesis gas make-up gas stream deficient in hydrogen with a trimming gas stream containing hydrogen to obtain a synthesis gas make-up gas stream enriched in hydrogen,

(c) a second mixing device for combining the synthesis gas make-up gas stream enriched in hydrogen with a synthesis gas cycle gas stream to obtain a synthesis gas feed gas stream,

(d) at least one methanol synthesis reactor, a conduit for introducing the synthesis gas feed gas stream into the at least one methanol synthesis reactor,

(e) a conduit for discharging a product gas stream containing methanol vapor and non-converted synthesis gas constituents from the at least one methanol synthesis reactor,

(f) a separating device for at least partly separating the methanol from the product gas stream by cooling and condensation,

(g) a fractionating device for fractionating the product gas stream after separation of the methanol into a synthesis gas cycle gas stream and a purge gas stream loaded with methanol residues, and a conduit for recirculating the synthesis gas cycle gas stream to the second mixing device,

(h) a washing device, a conduit for introducing the purge gas stream loaded with methanol residues into the washing device, a conduit for introducing the washing agent, a conduit for discharging a purge gas stream depleted of methanol from the washing device, and a conduit for discharging a washing agent loaded with methanol from the washing device,

(i) a hydrogen separation device, comprising a membrane separation stage and a pressure swing adsorption stage, a conduit for introducing the purge gas stream depleted of methanol into the hydrogen separation device, a conduit for discharging a gas stream enriched in hydrogen from the hydrogen separation device, and a conduit for discharging a gas stream depleted of hydrogen from the hydrogen separation device,

(j) a conduit for recirculating a gas stream enriched in hydrogen as trimming gas stream containing hydrogen to the first mixing device.

**12.** The plant according to claim 11, **characterized in that** in the hydrogen separation device the membrane separation stage and the pressure swing adsorption stage are connected in series and are in fluid connection with each other.

**13.** The plant according to claim 12, **characterized in that** in the hydrogen separation device first the membrane separation stage and then the pressure swing adsorption stage are traversed by the purge gas stream depleted of methanol.

**14.** The plant according to claim 13, furthermore comprising a conduit with which a first gas stream enriched in hydrogen, which is obtained as permeate in the membrane separation stage, is recirculated to the first mixing device as trimming gas stream.

**15.** The plant according to claim 14, furthermore comprising a conduit for introducing the retentate obtained in the membrane separation stage into the pressure swing adsorption stage and a conduit for introducing a second gas stream enriched in hydrogen, which is obtained in the pressure swing adsorption stage, as trimming gas stream into the first mixing device or for discharging from the plant as hydrogen export stream.

**16.** The plant according to claim 15, furthermore comprising a mixing device for combining the second gas stream enriched in hydrogen with the first gas stream enriched in hydrogen, and a conduit for supplying the mixed gas stream obtained to the first mixing device.

**17.** The plant according to claim 11, **characterized in that** the membrane separation stage and the pressure swing adsorption stage are connected in parallel in the hydrogen separation device, and furthermore comprising a conduit for introducing a part of the purge gas stream depleted of methanol into the membrane separation stage and a conduit for introducing the remaining part of the purge gas stream depleted of methanol into the pressure swing adsorption stage.

**18.** The plant according to claim 17, furthermore comprising a conduit with which a first gas stream enriched in hydrogen, which is obtained as permeate in the membrane separation stage, is recirculated to the first mixing device as trimming gas stream.

**19.** The plant according to claim 18, furthermore comprising a conduit for introducing a second gas stream enriched in hydrogen, which is obtained in the pressure swing adsorption stage, as trimming gas stream into the first mixing device or for discharging from the plant as hydrogen export stream.

**Revendications**

**1.** Procédé de fabrication de méthanol à partir d'un gaz de synthèse contenant de l'hydrogène, des oxydes de carbone et éventuellement des composants inertes, comprenant les étapes suivantes :

(a) la préparation d'un courant gazeux frais de gaz de synthèse contenant de l'hydrogène, des oxydes de carbone et éventuellement des composants inertes, qui contient un déficit d'hydrogène par rapport à la stoechiométrie prescrite pour la synthèse de méthanol,

(b) la réunion du courant gazeux frais de gaz de synthèse déficitaire en hydrogène avec un courant gazeux de compensation contenant de l'hydrogène pour former un courant gazeux frais de gaz de synthèse enrichi en hydrogène,

(c) la réunion du courant gazeux frais de gaz de synthèse enrichi en hydrogène avec le courant gazeux circulaire de gaz de synthèse obtenu à l'étape (g) pour former un courant gazeux d'alimentation de gaz de synthèse,

(d) l'introduction du courant gazeux d'alimentation de gaz de synthèse dans au moins un réacteur de synthèse de méthanol et la réaction catalytique au moins partielle des oxydes de carbone contenus dans le courant gazeux d'alimentation de gaz de synthèse avec de l'hydrogène dans des conditions de synthèse de méthanol pour former du méthanol,

(e) le déchargement d'un courant gazeux de produits contenant de la vapeur de méthanol et des constituants du gaz de synthèse non réagis de l'au moins un réacteur de synthèse de méthanol,

(f) la séparation au moins partielle du méthanol du courant gazeux de produits par refroidissement et condensation,

(g) la séparation du courant gazeux de produits après séparation du méthanol en un courant gazeux circulaire de gaz de synthèse et un courant gazeux de rinçage chargé avec des résidus de méthanol, le recyclage du courant gazeux circulaire de gaz de synthèse selon l'étape (c),

(h) l'introduction du courant gazeux de rinçage chargé avec des résidus de méthanol dans un dispositif de lavage, la mise en contact du courant gazeux de rinçage chargé avec des résidus de méthanol dans le dispositif de lavage avec un agent de lavage, de préférence de l'eau, le déchargement d'un courant gazeux de rinçage appauvri en méthanol et d'un agent de lavage chargé avec du méthanol du dispositif de lavage,

(i) l'introduction du courant gazeux de rinçage appauvri en méthanol dans un dispositif de séparation d'hydrogène, qui comprend un étage de séparation sur membrane et un étage d'adsorption modulée en pression, au moins un courant gazeux enrichi en hydrogène et au moins un courant gazeux appauvri en hydrogène étant obtenus,

(j) le recyclage d'au moins un courant gazeux enrichi en hydrogène en tant que courant gazeux de compensation contenant de l'hydrogène selon l'étape (b).

**2.** Procédé selon la revendication 1, **caractérisé en ce que**, dans le dispositif de séparation d'hydrogène, l'étage de séparation sur membrane et l'étage d'adsorption modulée en pression sont raccordés en série et se trouvent en connexion fluidique.

**3.** Procédé selon la revendication 2, **caractérisé en ce que**, dans le dispositif de séparation d'hydrogène, tout d'abord l'étage de séparation sur membrane, puis l'étage d'adsorption modulée en pression sont traversés par le courant gazeux de rinçage appauvri en méthanol.

**4.** Procédé selon la revendication 3, **caractérisé en ce que**, dans l'étage de séparation sur membrane, un premier courant gazeux enrichi en hydrogène est obtenu en tant que perméat, qui est réuni en tant que courant gazeux de compensation avec le courant gazeux frais de gaz de synthèse déficitaire en hydrogène.

**5.** Procédé selon la revendication 4, **caractérisé en ce que** le rétentat obtenu dans l'étage de séparation sur membrane est introduit dans l'étage d'adsorption modulée en pression et, dans l'étage d'adsorption modulée en pression, un deuxième courant gazeux enrichi en hydrogène est obtenu, qui est réuni en tant que courant gazeux de compensation avec le courant gazeux frais de gaz de synthèse déficitaire en hydrogène, ou déchargé du procédé en tant que courant d'exportation d'hydrogène.

**6.** Procédé selon la revendication 5, **caractérisé en ce que** le deuxième courant gazeux enrichi en hydrogène est tout d'abord réuni avec le premier courant gazeux enrichi en hydrogène, puis en tant que courant gazeux de compensation avec le courant gazeux frais de gaz de synthèse déficitaire en hydrogène.

**7.** Procédé selon la revendication 1, **caractérisé en ce que**, dans le dispositif de séparation d'hydrogène, l'étage de séparation sur membrane et l'étage d'adsorption modulée en pression sont raccordés en parallèle, une partie du courant gazeux de rinçage appauvri en méthanol étant introduit dans l'étage de séparation sur membrane et la partie restante du courant gazeux de rinçage appauvri en méthanol dans l'étage d'adsorption modulée en pression.

**8.** Procédé selon la revendication 7, **caractérisé en ce que**, dans l'étage de séparation sur membrane, un premier courant gazeux enrichi en hydrogène est obtenu en tant que perméat, qui est réuni en tant que courant gazeux de compensation avec le courant gazeux frais de gaz de synthèse déficitaire en hydrogène.

9. Procédé selon la revendication 7, **caractérisé en ce que**, dans l'étage d'adsorption modulée en pression, un deuxième courant gazeux enrichi en hydrogène est obtenu, qui est réuni en tant que courant gazeux de compensation avec le courant gazeux frais de gaz de synthèse déficitaire en hydrogène, ou déchargé du procédé en tant que courant d'exportation d'hydrogène.

10. Procédé selon l'une quelconque des revendications précédentes, **caractérisé en ce que** la préparation du courant gazeux frais de gaz de synthèse a lieu au moyen d'un étage de génération de gaz de synthèse, qui comprend un reformeur autotherme.

11. Unité pour la fabrication de méthanol à partir d'un gaz de synthèse contenant de l'hydrogène, des oxydes de carbone et éventuellement des composants inertes, comprenant les composants d'unité suivants :

   (a) un étage de génération d'un gaz de synthèse pour la préparation d'un courant gazeux frais de gaz de synthèse contenant de l'hydrogène, des oxydes de carbone et éventuellement des composants inertes, qui contient un déficit d'hydrogène par rapport à la stoechiométrie prescrite pour la synthèse de méthanol,
   (b) un premier dispositif de mélange pour la réunion du courant gazeux frais de gaz de synthèse déficitaire en hydrogène avec un courant gazeux de compensation contenant de l'hydrogène pour former un courant gazeux frais de gaz de synthèse enrichi en hydrogène,
   (c) un deuxième dispositif de mélange pour la réunion du courant gazeux frais de gaz de synthèse enrichi en hydrogène avec un courant gazeux circulaire de gaz de synthèse pour former un courant gazeux d'alimentation de gaz de synthèse,
   (d) au moins un réacteur de synthèse de méthanol, une conduite pour l'introduction du courant gazeux d'alimentation de gaz de synthèse dans l'au moins un réacteur de synthèse de méthanol,
   (e) une conduite pour le déchargement d'un courant gazeux de produits contenant de la vapeur de méthanol et des constituants du gaz de synthèse non réagis de l'au moins un réacteur de synthèse de méthanol,
   (f) un dispositif de séparation pour la séparation au moins partielle du méthanol du courant gazeux de produits par refroidissement et condensation,
   (g) un dispositif de séparation pour la séparation du courant gazeux de produits après séparation du méthanol en un courant gazeux circulaire de gaz de synthèse et un courant gazeux de rinçage chargé avec des résidus de méthanol, et une conduite pour le recyclage du courant gazeux circulaire de gaz de synthèse dans le deuxième dispositif de mélange,
   (h) un dispositif de lavage, une conduite pour l'introduction du courant gazeux de rinçage chargé avec des résidus de méthanol dans le dispositif de lavage, une conduite pour l'introduction de l'agent de lavage, une conduite pour le déchargement d'un courant gazeux de rinçage appauvri en méthanol du dispositif de lavage et une conduite pour le déchargement d'un agent de lavage chargé avec du méthanol du dispositif de lavage,
   (i) un dispositif de séparation d'hydrogène, comprenant un étage de séparation sur membrane et un étage d'adsorption modulée en pression, une conduite pour l'introduction du courant de gaz de rinçage appauvri en méthanol dans le dispositif de séparation d'hydrogène, une conduite pour le déchargement d'un courant gazeux enrichi en hydrogène du dispositif de séparation d'hydrogène et une conduite pour le déchargement d'un courant gazeux appauvri en hydrogène du dispositif de séparation d'hydrogène,
   (j) une conduite pour le recyclage d'un courant gazeux enrichi en hydrogène en tant que courant gazeux de compensation contenant de l'hydrogène dans le premier dispositif de mélange.

12. Unité selon la revendication 11, **caractérisée en ce que**, dans le dispositif de séparation d'hydrogène, l'étage de séparation sur membrane et l'étage d'adsorption modulée en pression sont raccordés l'un derrière l'autre et se trouvent en connexion fluidique.

13. Unité selon la revendication 12, **caractérisée en ce que**, dans le dispositif de séparation d'hydrogène, tout d'abord l'étage de séparation sur membrane, puis l'étage d'adsorption modulée en pression sont traversés par le courant gazeux de rinçage appauvri en méthanol.

14. Unité selon la revendication 13, comprenant en outre une conduite, avec laquelle un premier courant gazeux enrichi en hydrogène, qui est obtenu en tant que perméat dans l'étage de séparation sur membrane, est recyclé en tant que courant gazeux de compensation dans le premier dispositif de mélange.

**15.** Unité selon la revendication 14, comprenant en outre une conduite pour l'introduction du rétentat obtenu dans l'étage de séparation sur membrane dans l'étage d'adsorption modulée en pression et une conduite pour l'introduction d'un deuxième courant gazeux enrichi en hydrogène, qui est obtenu dans l'étage d'adsorption modulée en pression, en tant que courant gazeux de compensation dans le premier dispositif de mélange, ou pour le déchargement de l'unité en tant que courant d'exportation d'hydrogène.

**16.** Unité selon la revendication 15, comprenant en outre un dispositif de mélange pour la réunion du deuxième courant gazeux enrichi en hydrogène avec le premier courant gazeux enrichi en hydrogène, et une conduite pour l'introduction du courant gazeux mélangé ainsi obtenu dans le premier dispositif de mélange.

**17.** Unité selon la revendication 11, **caractérisée en ce que**, dans le dispositif de séparation d'hydrogène, l'étage de séparation sur membrane et l'étage d'adsorption modulée en pression sont raccordés en parallèle, et comprenant en outre une conduite pour l'introduction d'une partie du courant gazeux de rinçage appauvri en méthanol dans l'étage de séparation sur membrane et une conduite pour l'introduction de la partie restante du courant gazeux de rinçage appauvri en méthanol dans l'étage d'adsorption modulée en pression.

**18.** Unité selon la revendication 17, comprenant en outre une conduite avec laquelle un premier courant gazeux enrichi en hydrogène, qui est obtenu en tant que perméat dans l'étage de séparation sur membrane, est recyclé en tant que courant gazeux de compensation dans le premier dispositif de mélange.

**19.** Unité selon la revendication 18, comprenant en outre une conduite pour l'introduction d'un deuxième courant gazeux enrichi en hydrogène, qui est obtenu dans l'étage d'adsorption modulée en pression, en tant que courant gazeux de compensation dans le premier dispositif de mélange, ou pour le déchargement hors de l'unité en tant que courant d'exportation d'hydrogène.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0790226 B1
- DE 2934332 A1
- EP 1016643 A1
- US 20090018220 A1

**In der Beschreibung aufgeführte Nicht-Patentliteratur**

- Methanol'', Unterkapitel 5.2 ''Synthesis. Ullmann's Encyclopedia of Industrial Chemistry. Electronic Release, 1998